# EUROPÄISCHE PATENTSCHRIFT

(11) **EP 2 133 046 B1**
(45) Veröffentlichungstag und Bekanntmachung des Hinweises auf die Patenterteilung: **22.12.2010**
(21) Anmeldenummer: 09011974.4
(22) Anmeldetag: 27.11.2007
(51) Int. Cl.: A61F 5/058

(54) **Schiene zur Ruhigstellung eines Gelenks**
Rail for securing a joint
Rail destiné à l'immobilisation d'une articulation

(43) Veröffentlichungstag der Anmeldung: 16.12.2009
(62) Teilanmeldung aus: 07022933.1
(73) Patentinhaber: Kandt, Olaf, 25474 Bönningstedt (DE)
(72) Erfinder: Kandt, Olaf, 25474 Bönningstedt (DE)
(74) Vertreter: Seemann, Ralph

(56) Entgegenhaltungen:
- US-A- 4 840 168
- US-A- 5 772 620
- US-B1- 6 293 918

## Beschreibung

Die Erfindung betrifft eine Schiene zur Ruhigstellung eines Handgelenks, die am ruhig zu stellenden Handgelenk zu tragen ist, wobei die Schiene aus wenigstens einem ersten Flächenkörper besteht, der wenigstens eine Auflagefläche für einen Unterarm und eine Auflagefläche für einen Handballen aufweist, wobei ein Stützkörper, der aus einem flächigen Material besteht, vorgesehen ist, der in seiner Außenkontur eine Winkelstruktur aufweist, die mit der Unterseite der Auflageflächen verbindbar ist oder verbunden ist, wobei die Winkelstruktur zur Einstellung und Stützung eines Winkels zwischen der Auflagefläche für den Unterarm und der Auflagefläche für den Handballen ausgebildet ist.

Bei Beanspruchung des Muskelapparats des menschlichen Körpers kommt es im Alltag, am Arbeitsplatz und in der Freizeit immer wieder zu Verletzungen, die es notwendig machen, dass diese unter Einschluss eines oder mehrerer Gelenke ruhig gestellt werden, um eine komplikationslose Ausheilung zu gewährleisten.

Verletzungen der Knochen, Sehnen, Muskeln, Nerven oder Blutgefäße gehen oftmals mit großen Schmerzen einher. Dies gilt insbesondere für Knochenbrüche und Weichteilverletzungen. Symptome hierbei sind Bewegungs-, Anspannungs-, Druck- und Dehnschmerzen sowie Blutungen. Besonders häufig sind Verletzungen im Breiten- und Leistungssport sowie im Alltag. Eine Vielzahl von in diesen Bereichen auftretenden Verletzungen und Schmerzzuständen erfordert eine Schienenversorgung.

Bei leichten Verletzungen und Überlastsyndromen sind neben dem Ruhigstellen auch Kälteanwendungen sowie das Hochlagern des betroffenen Körperteils üblich. Weiterhin kommen ruhig stellende Tapeverbände, Salbenverbände und Zinkleimverbände sowie physikalische Therapien, Orthesen sowie Bandagenversorgungen zum Einsatz.

Bei schweren Verletzungen oder Schmerzzuständen werden ausschließlich Schienenverbände angelegt. Dabei wird der betroffene Körperteil unter Einschluss eines oder mehrerer Gelenke durch Einsatz einer Schiene oder Fertigorthese ruhig gestellt. Üblicherweise werden hierzu Schienen aus Materialien wie Naturgips, thermoplastischem Kunststoff, Glasfasern oder Polyester sowie Fertigorthesen aus Kunststoff oder Aluminium verwendet.

Fertigorthesen und individuell anzufertigende Schienen unterscheiden sich darin, dass die Ruhigstellung eines oder mehrerer Gelenke nach dem Anlegen einer individuell anzufertigenden Schiene eine Fixierung mit einer Binde erfordert. Fertigorthesen werden dagegen mit Klettverschlusssystemen ausgestattet, die zur Fixierung dienen. Aufgrund der aufwendigeren Herstellung werden Fertigorthesen aus Kostengründen ausschließlich bei Langzeitversorgungen angewendet. Bei kurzfristigen Anwendungen kommen Schienen zum Einsatz, die vom Therapeuten selbst hergestellt und individuell angepasst werden.

Die Schienenversorgung ist zeitaufwendig und kostenintensiv, da eine individuelle Anfertigung und Anpassung notwendig ist. Wegen des hohen Materialgewichts in verarbeitungsbedingter scharfkantiger Formung sind solche Schienen oftmals mit einem schlechten Tragekomfort zu tragen. Das Anlegen und Herstellen solcher Schienenverbände, wie z.B. das Formen des Gipses in die medizinisch gewünschte Schiene ohne scharfe Kanten, erfordern besondere Fähigkeiten und Kenntnisse, welche gezielt ausgebildet und trainiert werden müssen.

Mit solchen bekannten Schienensystemen ist es nicht möglich, schnell am Ort einer gerade erlittenen Verletzung eine Schiene anzulegen, mit der das betroffene Gelenk bzw. Körperteil ruhig gestellt werden kann, um eine weitere Verschlimmerung der Verletzung kurzfristig zu verhindern. Auch sind solche Schienen nicht einsetzbar, wenn noch eine Versorgung von die Haut betreffenden Verletzungen notwendig ist, da sich herkömmliche Schienensysteme nicht flexibel abnehmen lassen, um danach wieder angebracht zu werden.

Aus US 5 772 620 A ist eine Hand- und Handgelenkorthese mit einer flächigen, gepolsterten Auflage für Unterarm, Mittelhandknochen und Fingerglieder, mit einer Stützschiene und mit zwei Riemen zur Fixierung am Unterarm bekannt. Die Stützschiene umfasst mehrere Abschnitte, die jeweils in einem Winkel zueinander angeordnet sind. Ferner ist die Stützschiene flächig mit der Unterseite der Auflage verbunden.

Der Erfindung liegt daher die Aufgabe zugrunde, Schienen zur Ruhigstellung eines Gelenks oder Handgelenks, die am ruhig zu stellenden Gelenk oder Handgelenk zu tragen sind, zur Verfügung zu stellen, mit denen eine Sofortversorgung an einer Verletzung zur Ruhigstellung eines Gelenks erfolgen kann und die gleichzeitig einen erhöhten Tragekomfort bieten sowie kostengünstig herstellbar und konfektionierbar sind.

Die der Erfindung zugrunde liegende Aufgabe wird gelöst durch eine Schiene zur Ruhigstellung eines Handgelenks, die am ruhig zu stellenden Handgelenk zu tragen ist, wobei die Schiene aus wenigstens einem ersten Flächenkörper besteht, der wenigstens eine Auflagefläche für einen Unterarm und eine Auflagefläche für einen Handballen aufweist, wobei ein Stützkörper, der aus einem flächigen Material besteht, vorgesehen ist, der in seiner Außenkontur eine Winkelstruktur aufweist, die mit der Unterseite der Auflageflächen verbindbar ist oder verbunden ist, wobei die Winkelstruktur zur Einstellung und Stützung eines Winkels zwischen der Auflagefläche für den Unterarm und der Auflagefläche für den Handballen ausgebildet ist, die dadurch weitergebildet ist, dass der Stützkörper beidseitig seiner Längsachse Falzlinien aufweist, um die ein langer Teil des Stützkörpers in eine U-Form bringbar ist, wobei das flächige Material des Stützkörpers in eine dreidimensionale Form gebogen oder gefaltet ist, die eine Konturseite aufweist, die mit der Rückseite der Auflageflächen des Flächenkörpers verbindbar ist oder verbunden ist.

Mit der so ausgebildeten Schiene ist es möglich, ein Handgelenk ruhig zu stellen, wobei der Handballen bzw. die Hand in einem das Gelenk entlastenden Winkel zum Unterarm ruhig gestellt wird. Auch die erfindungsgemäße Schiene für ein Handgelenk ist aus einem Flächenkörper aufgebaut, der mit wenigen Handgriffen in eine stabile Schiene faltbar bzw. biegbar ist. Da diese Form der Schiene nicht, wie eine Schlaufe, selbst stabilisierend ist, ist ein Stützkörper vorgesehen, der der Struktur von Auflageflächen den nötigen Halt vermittelt.

Vorzugsweise besteht der Stützkörper aus einem flächigen Material. Vorzugsweise ist das flächige Material des Stützkörpers in eine dreidimensionale Form gebogen oder gefaltet, die eine Konturseite aufweist, die mit der Rückseite der Auflageflächen des Flächenkörpers verbindbar ist oder verbunden ist. Die Verbindung ist dabei vorteilhafterweise eine Klebverbindung, eine Steckverbindung oder eine Verbindung mittels Klammern, insbesondere Heftklammern.

Sowohl für die erfindungsgemäße Schiene in Form einer Schlaufe als auch die erfindungsgemäße Schiene für ein Handgelenk besteht in einer vorteilhaften Ausbildung der Flächenkörper und/oder der Stützkörper aus einem zugfesten Material. Unter einem zugfesten Material wird im Rahmen der Erfindung ein Material verstanden, das gegenüber Zugbelastungen in der Fläche des Flächenkörpers resistent ist und nicht nachgibt. So erhalten die Schlaufe bzw. der Stützkörper und die Auflageflächen, insbesondere entlang der Biegungen bzw. Faltungen und Falzlinien ihre Festigkeit und Stabilität.

Eine besonders einfache Ausführungsform besteht darin, dass der Flächenkörper und/oder der Stützkörper aus Pappe oder Karton bestehen, insbesondere aus Wellpappe. Diese Materialien sind in ihrer flächigen Ebene zugfest, jedoch um Falzlinien gut biegbar. Andere Materialien, wie Kunststoffe, die in einer Richtung senkrecht zu ihrer Fläche elastischer sind, können auch ohne Falzlinien gebogen werden.

In der Ausführungsform der Flächenkörper oder Stützkörper als Wellpappe sind die innen liegenden Rippen der Wellpappe vorzugsweise wenigstens teilweise orthogonal zur Biegung des Flächenkörpers bzw. des Stützkörpers oder wenigstens teilweise parallel zu den Falzlinien des Flächenkörpers und/oder des Stützkörpers ausgerichtet. Damit sind die Rippen wenigstens teilweise entlang des Rückens einer Biegung des Flächenkörpers ausgerichtet und unterstützen die stabilisierende Wirkung der Biegung. Wellpappe ist in der Richtung der Rippen gegen Verbiegung besonders stabil.

In einer vorteilhaften Ausführungsform weist der Flächenkörper der Schiene zur Ruhigstellung eines Handgelenks seitlich der Auflagefläche für den Unterarm Abschnitte auf, die, insbesondere um parallel zur Richtung des Unterarms angeordnete Falzlinien, um einen Unterarm biegbar sind. So erhält die Schiene zusätzliche Stabilität und eine stärkere Verankerung am Unterarm, mit dem die Schiene verbunden wird.

Weiter vorteilhaft weist der Flächenkörper wenigstens eine gegenüber der Auflagefläche für den Handballen anwinkelbare Auflagefläche für Finger und/oder eine Auflagefläche für Daumen auf. Mit diesen Auflageflächen können auch die Finger bzw. Daumen ruhig gestellt werden und sind gegen Verletzungen geschützt. Vorzugsweise ist die Auflagefläche für Finger zu einer 4-Fingerschiene, einer 3-Fingerschiene, einer 2-Fingerschiene oder einer 1-Fingerschiene zuschneidbar ist oder vollständig entfernbar. Im letzteren Fall wird eine reine Handgelenkschiene erhalten.

Der Flächenkörper und der Stützkörper können aus zwei verschiedenen Elementen zusammengesetzt werden. Eine vorteilhafte Ausbildung besteht jedoch auch darin, dass der Flächenkörper und der Stützkörper aus einem einstückigen Flächenkörper bestehen. So sind bereits alle Elemente der Schiene in einem Körper vorhanden, was eine besonders einfache und kostengünstige Maßnahme darstellt.

Vorzugsweise ist die Schiene symmetrisch zur Anwendung an einem Gelenk oder Handgelenk sowohl eines linken als auch eines rechten Körperteils ausgebildet.

Die erfindungsgemäßen Schienen sind beispielsweise durch Umwickeln mit Verbandmaterial an den ruhig zu stellenden Gelenken bzw. den angrenzenden Körperteilen zu befestigen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:
- Fig. 1: eine erfindungsgemäße Handgelenksschiene im ausge- klappten Zustand,
- Fig. 2: eine weitere erfindungsgemäße Handgelenksschiene im ausgeklappten Zustand in schematischer Darstellung,
- Fig. 3: eine weitere erfindungsgemäße Handgelenksschiene im ausgeklappten Zustand in schematischer Darstellung,
- Fig. 4: eine schematische Seitenansicht einer erfindungsge- mäßen Handgelenksschiene und
- Fig. 5: eine schematische Rückansicht einer erfindungsgemä- ßen Handgelenksschiene.

In den folgenden Figuren sind jeweils gleiche oder gleichartige Elemente bzw. entsprechende Teile mit denselben Bezugsziffern versehen, so dass von einer entsprechenden erneuten Vorstellung abgesehen wird.

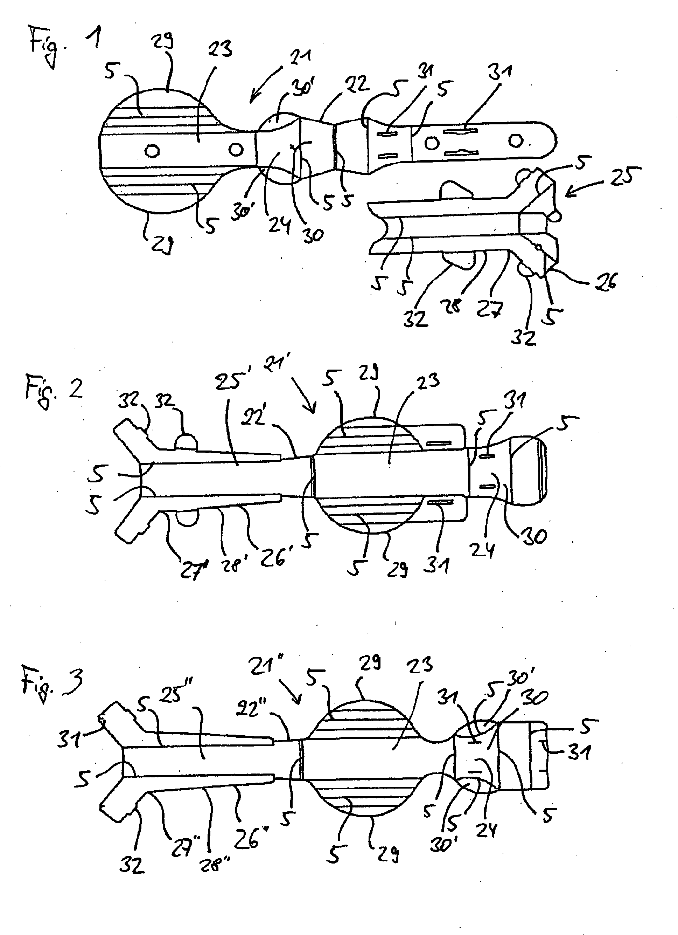

In den Fig. 1, 2 und 3 sind jeweils die Flächenkörper 22, 22', 22" von drei verschiedenen Ausführungsbeispielen von erfindungsgemäßen Schienen 21, 21', 21" zur Ruhigstellung von Handgelenken schematisch im aufgeklappten Zustand dargestellt. Darin sind jeweils die äußeren Konturen, Falzlinien und Öffnungen bzw. Schlitze sichtbar.

Das Ausführungsbeispiel gemäß Fig. 1 besteht aus zwei Flächenkörpern, nämlich einem Flächenkörper 22, der eine Auflagefläche 23 für einen Unterarm und eine Auflagefläche 24 für einen Handballen aufweist, sowie einem Stützkörper 25. Der Flächenkörper 22 weist außerdem biegbare Abschnitte 29 mit entsprechenden Falzlinien 5 auf, die um einen Unterarm biegbar sind, so dass eine schonende und sichere Befestigung der Schiene 21 an einem Unterarm möglich ist.

Zu beiden Seiten der Auflagefläche 24 für Handballen sind Auflageflächen 30' für Daumen vorgesehen. Somit ist dieses Ausführungsbeispiel beidseitig nutzbar. An die Auflagefläche 24 für einen Handballen schließt sich eine Auflagefläche 30 für Finger an.

An die Auflagefläche 30 für Finger schließt sich eine Falzlinie 5 an, an die sich ein zweiter Teil des Flächenkörpers 22 anschließt, der zu den Unterseiten der Auflageflächen 23, 24, 30 klappbar ist. Die Falzlinien 5 dieses zweiten Teils entsprechen dabei den Falzlinien 5 zwischen den Auflageflächen 23, 24, 30. Der zweite Teil des Flächenkörpers 22 weist Öffnungen 31 für Steckverbindungen auf. In diese Öffnungen 31 greifen Steckelemente 32 oder Nasen für Steckverbindungen ein, die am Stützkörper 25 angeordnet sind. Der Stützkörper 25 weist beidseitig seiner Längsachse Falzlinien 5 auf, um die der lange Teil des Stützkörpers 25 in eine U-Form bringbar ist.

Die Außenkontur 26 des Stützkörpers 25 weist außerdem eine Winkelstruktur 27 auf, die zusammen mit der Konturseite 28 des Stützkörpers 25 in Verbindung bringbar ist mit der Unterseite des Flächenkörpers 22. Der durch den Stützkörper 25 eingestellte Winkel beträgt etwa 45°. In diesem Winkel werden die Auflagefläche 23 für einen Unterarm und die Auflagefläche 24 für einen Handballen zueinander eingestellt.

In den Fig. 1, 2 und 3 ist die Schiene 21, 21', 21" jeweils so orientiert, dass ein Unterarm von links auf der Auflagefläche 23 für einen Unterarm gelagert wird und der Handballen rechts auf der Auflagefläche 24 für den Handballen aufsetzt. Für eine optimale Verstärkung ist die Rippenstruktur bei Ausführungen mit Wellpappe vorzugsweise parallel zur Längsachse der Schiene 21, 21', 21" ausgerichtet.

In Fig. 2 ist ein Ausführungsbeispiel einer erfindungsgemäßen Schiene 21' für ein Handgelenk dargestellt, die aus einem gemeinsamen Flächenkörper 22' besteht, der bereits einen Stützkörper 25' aufweist. Der nach unten klappbare Teil, der den Stützkörper 25' beinhaltet, ist entlang Falzlinien 5 in eine U-Form bringbar. Die Konturseite 28' des Stützkörpers 25' weist Nasen bzw. Steckelemente 32 für Steckverbindungen auf, die mit Öffnungen 31 in Eingriff gebracht werden.

Das Ausführungsbeispiel in Fig. 3 entspricht im Wesentlichen dem Ausführungsbeispiel gemäß Fig. 2. Im Unterschied zu dem in Fig. 2 gezeigten Beispiel weist das Ausführungsbeispiel in Fig. 3 keine Öffnungen und Nasen auf, sondern Schlitze 31, die miteinander in Eingriff gebracht werden. In allen Beispielen sind einfach ausgeführte Steckverbindungen verwirklicht, die eine ausreichende Stabilität der jeweiligen Schienen 21, 21', 21" aufweisen.

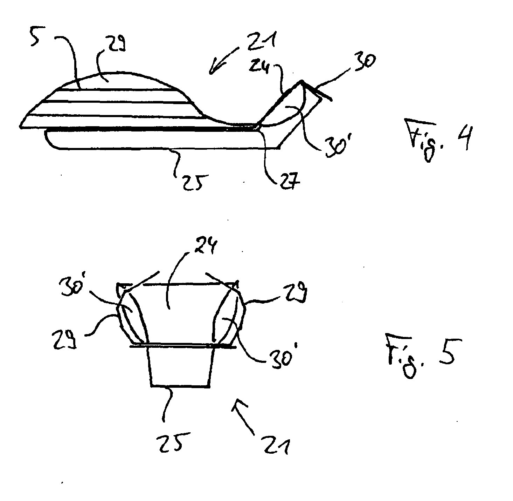

In den Fig. 4 und 5 sind schematische Darstellungen des Ausführungsbeispiels aus Fig. 1 in einer Seitenansicht bzw. einer Rückansicht dargestellt. Fig. 4 zeigt eine schematische Seitenansicht der zusammengesetzten Schiene 21 mit einem winkelförmigen Stützkörper 25, der eine Winkelstruktur 27 aufweist, sowie einem Auflageflächenkörper 22, der einen biegbaren Abschnitt 29 mit entsprechenden Falzlinien 5 für den Unterarm aufweist sowie einen Abschnitt 24 für den Handballen sowie biegbare Auflageflächen 30 für Finger und 30' für einen Daumen.

In Fig. 5 ist das gleiche Ausführungsbeispiel aus einer Perspektive des Unterarms dargestellt. Die U-Form des Stützkörpers 25 ist erkennbar sowie die Tatsache, dass die biegbaren Abschnitte 29 für den Unterarm in Form eines Unterarms gebogen sind. Die Auflagefläche für den Handballen 24 weist an den Seiten eine leicht gebogene Struktur auf, wodurch eine einfache Lagerung des Daumens möglich ist.

### Bezugszeichenliste

- 5: Falzlinien
- 21, 21', 21": Schiene
- 22, 22', 22": Flächenkörper
- 23: Auflagefläche für Unterarm
- 24: Auflagefläche für Handballen
- 25, 25', 25": Stützkörper
- 26, 26', 26": Außenkontur des Stützkörpers
- 27, 27', 27": Winkelstruktur
- 28, 28', 28": Konturseite des Stützkörpers
- 29: biegbarer Abschnitt für Unterarm
- 30: Auflagefläche für Finger
- 30': Auflagefläche für Daumen
- 31: Öffnungen und Schlitze für Steckverbindungen
- 32: Steckelemente für Steckverbindungen

## Patentansprüche

1. Schiene (21, 21', 21") zur Ruhigstellung eines Handgelenks, die am ruhig zu stellenden Handgelenk zu tragen ist, wobei die Schiene (21, 21', 21") aus wenigstens einem ersten Flächenkörper (22, 22', 22") besteht, der wenigstens eine Auflagefläche (23) für einen Unterarm und eine Auflagefläche (24) für einen Handballen aufweist, wobei ein Stützkörper (25, 25', 25"), der aus einem flächigen Material besteht, vorgesehen ist, der in seiner Außenkontur (26, 26', 26") eine Winkelstruktur (27, 27', 27") aufweist, die mit der Unterseite der Auflageflächen (23, 24) verbindbar ist oder verbunden ist, wobei die Winkelstruktur (27, 27', 27") zur Einstellung und Stützung eines Winkels zwischen der Auflagefläche (23) für den Unterarm und der Auflagefläche (24) für den Handballen ausgebildet ist, **dadurch gekennzeichnet, dass** der Stützkörper (25, 25', 25") beidseitig seiner Längsachse Falzlinien (5) aufweist, um die ein langer Teil des Stützkörpers (25, 25', 25") in eine U-Form bringbar ist, wobei das flächige Material des Stützkörpers (25, 25', 25") in eine dreidimensionale Form gebogen oder gefaltet ist, die eine Konturseite (28, 28', 28") aufweist, die mit der Rückseite der Auflageflächen (23, 24) des Flächenkörpers (22, 22', 22") verbindbar ist oder verbunden ist.

2. Schiene (21, 21', 21") nach Anspruch 1, **dadurch gekennzeichnet, dass** die Verbindung eine Klebverbindung, eine Steckverbindung oder eine Verbindung mittels Klammern, insbesondere Heftklammern, ist.

3. Schiene (1; 21, 21', 21") nach Anspruch 1 oder 2, **dadurch gekennzeichnet, dass** der Flächenkörper (2; 22, 22', 22") und/oder der Stützkörper (25, 25', 25") aus einem zugfesten Material bestehen.

4. Schiene (1; 21, 21', 21") nach einem der Ansprüche 1 bis 3, **dadurch gekennzeichnet, dass** der Flächenkörper (2; 22, 22', 22") und/oder der Stützkörper (25, 25', 25") aus Pappe oder Karton bestehen.

5. Schiene (1; 21, 21', 21") nach Anspruch 4, **dadurch gekennzeichnet, dass** der Flächenkörper (2; 22, 22', 22") und/oder der Stützkörper (25, 25', 25") aus Wellpappe besteht.

6. Schiene (1; 21, 21', 21") nach Anspruch 5, **dadurch gekennzeichnet, dass** innen liegende Rippen (12) der Wellpappe wenigstens teilweise orthogonal zur Biegung des Flächenkörpers (2; 22, 22', 22") bzw. des Stützkörpers (25, 25', 25") oder wenigstens teilweise parallel zu den Falzlinien (5) des Flächenkörpers (2; 22, 22', 22") und/oder des Stützkörpers (5) ausgerichtet sind.

7. Schiene (21, 21', 21") nach einem der Ansprüche 1 bis 6, **dadurch gekennzeichnet, dass** der Flächenkörper (22, 22', 22") seitlich der Auflagefläche (23) für den Unterarm Abschnitte (29) aufweist, die, insbesondere um parallel zur Richtung des Unterarms angeordnete Falzlinien (5), um einen Unterarm biegbar sind.

8. Schiene (21, 21', 21") nach einem der Ansprüche 1 bis 7, **dadurch gekennzeichnet, dass** der Flächenkörper (22, 22', 22") wenigstens eine gegenüber der Auflagefläche (24) für den Handballen anwinkelbare Auflagefläche (30) für Finger und/oder eine Auflagefläche (30') für Daumen aufweist.

9. Schiene (21, 21', 21") nach Anspruch 8, **dadurch gekennzeichnet, dass** die Auflagefläche (30) für Finger zu einer 4-Fingerschiene, einer 3-Fingerschiene, einer 2-Fingerschiene oder einer 1-Fingerschiene zuschneidbar ist oder vollständig entfernbar ist.

10. Schiene (21, 21', 21") nach einem der Ansprüche 1 bis 9, **dadurch gekennzeichnet, dass** der Flächenkörper (22, 22', 22") und der Stützkörper (25, 25', 25") aus einem einstückigen Flächenkörper bestehen.

11. Schiene (1; 21, 21', 21") nach einem der Ansprüche 1 bis 10, **dadurch gekennzeichnet, dass** die Schiene (1; 21, 21', 21") symmetrisch zur Anwendung an einem Gelenk sowohl eines linken als auch eines rechten Körperteils ausgebildet ist.

## Claims

1. A splint (21, 21', 21") for immobilising a wrist, which is to be worn on the wrist to be immobilised, wherein the splint (21, 21', 21") consists of at least one first flat body (22, 22', 22"), which has at least one engagement surface (23) for an underarm and an engagement surface (24) for a heel of the hand, wherein a support body (25, 25', 25") is provided, which consists of a laminar material and whose outer contour (26, 26', 26") has an angular structure (27, 27', 27"), which is connectable or connected to the underside of the engagement surfaces (23, 24), wherein the angular structure (27, 27', 27") is constructed to set and support an angle between the engagement surface (23) for the underarm and the engagement surface (24) for the heel of the hand, **characterised in that** the support body (25, 25', 25") has fold lines (25) on both sides of its longitudinal axis, about which a long portion of the support body (25, 25, 25") is moveable into a U shape, wherein the laminar material of the support body (25, 25', 25") is bent or folded into a three dimensional shape, which has a contour side (28, 28', 28"), which is connectable or connected to the rear side of the engagement surfaces (23, 24) of the flat body (22, 22', 22").

2. A splint (21, 21', 21") as claimed in claim 1, **characterised in that** the connection is an adhesive connection, a plug connection or a connection by means of clips, particularly staples.

3. A splint (1; 21, 21', 21") as claimed in claim 1 or 2, **characterised in that** the flat body (2; 22, 22', 22") and/or the support body (25, 25', 25") consist of a tension-resistant material.

4. A splint (1; 21, 21', 21") as claimed in one of claims 1 to 3, **characterised in that** the flat body (2; 22, 22', 22") and/or the support body (25, 25', 25") consist of cardboard or card.

5. A splint (1; 21, 21', 21") as claimed in claim 4, **characterised in that** the flat body (2; 22, 22', 22") and/or the support body (25, 25', 25") consist of corrugated cardboard.

6. A splint (1; 21, 21', 21") as claimed in claim 5, **characterised in that** internal ribs (12) of the corrugated cardboard are aligned at least in part orthogonally to the bending of the flat body (2; 22, 22', 22") or of the support body (25, 25', 25") or at least in part parallel to the fold lines (5) of the flat body (2; 22, 22', 22") and/or of the support body (5).

7. A splint (21, 21', 21") as claimed in one of claims 1 to 6, **characterised in that** laterally of the engagement surface (23) for the underarm, the flat body (2; 22, 22', 22") has sections (29), which are bendable around an underarm, particularly about fold lines (5) disposed parallel to the direction of the underarm.

8. A splint (21, 21', 21") as claimed in one of claims 1 to 7, **characterised in that** the flat body (22, 22', 22") has at least one engagement surface (30) for fingers, which may be angled with respect to the engagement surface (24) for the heel of the hand, and/or an engagement surface (30') for thumbs.

9. A splint (21, 21', 21") as claimed in claim 8, **characterised in that** the engagement surface (30) for fingers may be cut to size to form a 4-finger splint, a 3-finger splint, a 2-finger splint or a 1-finger splint or is completely removable.

10. A splint (21, 21', 21") as claimed in one of claims 1 to 9, **characterised in that** the flat body (22, 22', 22") and the support body (25, 25', 25") consist of a one-piece flat body.

11. A splint (1; 21, 21', 21") as claimed in one of claims 1 to 10, **characterised in that** the splint (1; 21, 21', 21") is of symmetrical construction for use on a joint of both a left hand and also a right hand body portion.

## Revendications

1. Attelle (21, 21', 21") pour immobiliser un poignet, qui doit être portée sur un poignet à immobiliser, l'attelle (21, 21', 21") étant constituée d'au moins un premier corps plan (22, 22', 22") comprenant au moins une surface d'appui (23) pour un avant-bras et une surface d'appui (24) pour le talon de la main, l'attelle comprenant un corps d'appui (25, 25', 25") constitué d'un matériau plan comportant, au niveau de son contour extérieur (26, 26', 26"), une structure angulaire (27, 27', 27") qui peut être assemblée à la partie inférieure de la surface d'appui (23, 24) ou qui est assemblée à celle-ci, la structure angulaire (27, 27', 27") étant destinée à l'ajustement et au renforcement d'un angle entre la surface d'appui (23) pour l'avant-bras et la surface d'appui (24) pour le talon de la main, **caractérisée en ce que** le corps d'appui (25, 25', 25") comprend des lignes de pliage (5) des deux côtés de son axe longitudinal, autour de chacune desquelles peut être pliées une partie allongée du corps d'appui (25, 25', 25") en forme de U, le matériau plan du corps d'appui (25, 25', 25") étant courbé ou plié selon une configuration tridimensionnelle présentant un côté formant contour (28, 28', 28") pouvant être assemblé à la partie arrière de la surface d'appui (23, 24) du corps plan (22, 22', 22"), ou étant assemblé à celle-ci.

2. Attelle (21, 21', 21") selon la revendication 1, **caractérisée en ce que** l'assemblage est un assemblage collé, un assemblage par emboîtement ou un assemblage au moyen d'attaches, notamment d'agrafes.

3. Attelle (1 ; 21, 21', 21") selon la revendication 1 ou la revendication 2, **caractérisée en ce que** le corps plan (2 ; 22, 22', 22") et / ou le corps d'appui (25, 25', 25") sont constitués d'un matériau résistant à la traction.

4. Attelle (1 ; 21, 21', 21") selon l'une des revendications 1 à 3, **caractérisée en ce que** le corps plan (2 ; 22, 22', 22") et / ou le corps d'appui (25, 25', 25") sont constitués de carton ou de carton fort.

5. Attelle (1 ; 21, 21', 21") selon la revendication 4, **caractérisée en ce que** le corps plan (2 ; 22, 22', 22") et / ou le corps d'appui (25, 25', 25") sont constitués de carton ondulé.

6. Attelle (1 ; 21, 21', 21") selon la revendication 5, **caractérisée en ce que** des nervures intérieures (12) du carton ondulé sont orientées au moins pour partie orthogonalement à la courbure du corps plan (2 ; 22, 22', 22") ou bien du corps d'appui (25, 25', 25") ou au moins pour partie parallèlement aux lignes de pliage (5) du corps plan (2 ; 22, 22', 22") et / ou du corps d'appui (25, 25', 25").

7. Attelle (21, 21', 21") selon l'une des revendications 1 à 6, **caractérisée en ce que** le corps plan (22, 22', 22") comprend, du côté de la surface d'appui (23), des sections (29) pour l'avant-bras qui peuvent être recourbées autour d'un avant-bras, en particulier autour des lignes de pliage (5) parallèlement à la direction de l'avant-bras.

8. Attelle (21, 21', 21") selon l'une des revendications 1 à 7, **caractérisée en ce que** le corps plan (22 22', 22') comprend au moins une surface d'appui (30) pour les doigts et / ou une surface d'appui (30') pour les pouces, au niveau de la surface d'appui (24) pour le talon de la main.

9. Attelle (21, 21', 21") selon la revendication 8, **caractérisée en ce que** la surface d'appui (30) pour les doigts peut être facilement coupée à la taille d'une attelle pour quatre doigts, d'une attelle pour trois doigts, d'une attelle pour deux doigts ou d'une attelle pour un seul doigt, ou est totalement amovible.

10. Attelle (21, 21', 21") selon l'une des revendications 1 à 9, **caractérisée en ce que** le corps plan (22, 22', 22") et le corps d'appui (25, 25', 25") sont constitués d'un corps plan en une seule pièce.

11. Attelle (1 ; 21, 21', 21") selon l'une des revendications 1 à 10, **caractérisée en ce que** l'attelle (1 ; 21, 21', 21") est réalisée symétrique, en vue d'être utilisée sur une articulation tant d'un membre gauche que d'un membre droit.
